# EUROPEAN PATENT APPLICATION

(11) **EP 3 144 004 A1**
(43) Date of publication of application: **22.03.2017**
(21) Application number: 16196181.8
(22) Date of filing: 06.10.2010
(51) Int. Cl.: A61K 35/66, A01N 63/00, A01N 25/00, A61K 31/43, A61K 35/74, A61K 45/06, A61K 9/00, A61K 9/16, A61K 9/48, A61P 1/12, A61P 31/04

(54) **ANTIBIOTIC FORMULATIONS PROVIDING REDUCED GASTROINTESTINAL SIDE EFFECTS AND CLOSTRIDIUM DIFFICILE INFECTION RELAPSE, AND RELATED METHODS**

(30) Priority: 06.10.2009 US 249144 P
(62) Divisional of application: 10822585.5
(71) Applicant: Dorfner, Scott, Moorestown, NJ 08057 (US)
(72) Inventor: Dorfner, Scott, Moorestown, NJ 08057 (US)
(74) Representative: Petraz, Gilberto Luigi

(57) **Abstract**

The invention includes a formulation comprising: (i) a therapeutically effective amount of at least one antibiotic; and (ii) a therapeutically effective amount of at least one probiotic material. The formulation is prepared in a dosage form for delivery to the gastrointestinal tract. The invention further includes a method of preventing or minimizing the proliferation of *Clostridium difficile* in the gastrointestinal tract of a mammal undergoing an antibiotic therapy comprising by administration of the formulation of the invention for the duration of the antibiotic therapy; methods of preventing or minimizing the occurrence of antibiotic-associated diarrhea in a mammal undergoing an antibiotic therapy that includes administering the formulation; and methods of preventing or minimizing the occurrence of antibiotic-associated colitis or pseudomembranous colitis in a patient undergoing an antibiotic therapy that includes administering the formulation.

Also included are methods of reducing or preventing a failure of treatment for an antibiotic-treatable infection in a patient comprising orally administering the formulation.

Described by the invention are formulations for the treatment of a C. *difficile* infection comprising: (i) a therapeutically effective amount of at least one antibiotic; and (ii) a therapeutically effective amount of at least one probiotic material. The antibiotic includes a non-systemic gram negative antibiotic (such as, for example, fodaximicin) and formulation is prepared in a dosage form for delivery to the gastrointestinal tract. Also included are methods of treatment of a C. *difficile* infection in mammal that include administering to the digestive tract of the mammal the above described formulation. Such methods provide that the likelihood that the mammal shall experience a reoccurrence of the C. *difficile* infection is 70% or less.

## Description

### CROSS REFERENCE TO RELATED APPLICATION

This application claims priority under 35 U.S.C § 119 to United States Provisional Patent Application serial no. 61/249,144, filed October 6, 2009, the contents of which are incorporated herein by reference.

### BACKGROUND OF THE INVENTION

Bacterial infections in humans and other mammals are commonly treated using antibiotics. Antibiotic therapy can be life-saving, depending on the nature and type of infection. When used to treat less significant infections, can vastly improve the quality of life of a patient by facilitating a rapid recovery from an infection. However, in many patients, use of antibiotics is accompanied by a side effect of gastrointestinal distress, ranging from the mild (upset stomach, diarrhea) to the severe (pseudomembranous colitis). The side effect of diarrhea in the pediatric population undergoing treatment with antibiotics is reported as significant, resulting in treatment failures (for noncompliance with the prescribed antibiotic regimen) and significant gastrointestinal side effects according to the American Academy of Pediatrics. Similarly, the American Academy of Geriatrics has determined that antibiotic-associated diarrhea and the development of potentially drug-resistant strains (as a consequence of treatment failures) constitutes a direct and significant health threat to the over 65 age group.

Gastrointestinal side effects are reported in the literature to arise from the elimination of the beneficial flora necessary to facilitate normal digestive processes of the gut by the antibiotics. Specifically, it is believed that the use of antibiotics results in disruption of the normal ecology of the gut, causing a decrease in the population of beneficial bacteria and an abnormal and harmful increase in less beneficial or harmful bacteria, particularly *Clostridium difficile.*

*Clostridium difficile* is reported as the primary cause of antibiotic-associated diarrhea and colitis. In healthy, non-antibiotic treated individuals, *C*. *difficile* is found primarily in the large intestine in minute amounts in about 5% of the adult population. In such healthy individuals, the *C*. *difficile* population is maintained in check by the beneficial bacterial population of the intestine. In patients treated with antibiotics, the over proliferation of *C*. *difficile* can cause diarrhea, ranging from a mild disturbance to a very severe illness with ulceration and bleeding from the colon (pseudomembranous colitis) and, at worst, perforation of the intestine leading to peritonitis. It can be fatal.

Over-proliferation of *C*. *difficile* can occur even in individuals who do not carry the bacteria in their normal gut flora when such individuals re exposed to *C*. *difficile* or its spores by, for example, contact with the feces of a carrier. Such cross-contamination occurs readily in numerous common settings, such as nursing homes, assisted living facilities, hospitals, day care and other medical and care facilities. Most of those affected are elderly patients some with fragile health or other underlying illnesses and younger patients, with less well developed immune systems.

Attempts have been made to counter the cidal effects to beneficial bacteria of an antibiotic by advising antibiotic-consuming patients to ingest probiotic nutritional supplements. These attempts are infeasible and practically disadvantageous means of addressing the problems of side effects, since the antibiotic and the supplement are seldom ingested contemporaneously and patients generally fail to comply with the two-step regimen. These disadvantages are particularly exacerbated in young children and the elderly, the populations that suffer most frequently and significantly from antibiotic-related side effects.

In addition, for those individuals that do develop *C*. *difficile* infections, there have been some attempts to identify antibiotic therapies that act on the *C*. *difficile.* Such attempts have been limitedly successful. While the patients treated experienced a reduction in infection when on the course of *C*. *difficile*-targeted antibiotics, those patients are prone to a reoccurrence of the infection upon completion of the course.

There remains a need in the art to address the above-described side effect associated with antibiotic therapy that is effective, practical and facilitates patient compliance and, for those patients who do develop a *C*. *difficile* infection, an effective therapy that reduces the likelihood of reoccurrence.

### BRIEF SUMMARY OF THE INVENTION

The invention includes a formulation comprising: (i) a therapeutically effective amount of at least one antibiotic; and (ii) a therapeutically effective amount of at least one probiotic material. The formulation is prepared in a dosage form for delivery to the gastrointestinal tract.

The invention further includes a method of preventing or minimizing the proliferation of *Clostridium difficile* in the gastrointestinal tract of a mammal undergoing an antibiotic therapy comprising by administration of the formulation of the invention for the duration of the antibiotic therapy; methods of preventing or minimizing the occurrence of antibiotic-associated diarrhea in a mammal undergoing an antibiotic therapy that includes administering the formulation; and methods of preventing or minimizing the occurrence of antibiotic-associated colitis or pseudomembranous colitis in a patient undergoing an antibiotic therapy that includes administering the formulation.

Also included are methods of reducing or preventing a failure of treatment for an antibiotic-treatable infection in a patient comprising orally administering the formulation.

Described by the invention are formulations for the treatment of a *C*. *difficile* infection comprising: (i) a therapeutically effective amount of at least one antibiotic; and (ii) a therapeutically effective amount of at least one probiotic material. The antibiotic includes a non-systemic gram negative antibiotic (such as, for example, fodaximicin) and formulation is prepared in a dosage form for delivery to the gastrointestinal tract.

Also included are methods of treatment of a *C*. *difficile* infection in mammal that include administering to the digestive tract of the mammal the above described formulation. Such methods provide that the likelihood that the mammal shall experience a reoccurrence of the *C*. *difficile* infection is 70% or less.

### DETAILED DESCRIPTION OF THE INVENTION

Unexpectedly, it has been found that the combination of a probiotic material(s) and one or more antibiotics in a single dosage form results in no significant diminishment of the effectiveness of the antibiotic(s), or alteration of the pharmacokinetics of the antibiotic. Additionally, patients (mammalian, especially human) undergoing a course of antibiotic therapy using the formulations of the invention do not experience any significant gastrointestinal distress, diarrhea, and/or colitis, such as is commonly experienced with other prior art antibiotic formulations.

The invention may include formulations for oral administration that include at least one antibiotic and at least one probiotic material. Any antibiotics, known or to be discovered in the art may be used. Suitable antibiotics include those of the classes aminoglycosides, ansamycins, carbacephmes, cephalosporins, glycopeptides, macrolides, penicillins, polypeptides, quinolones, sulfonamides, tetracyclines and mixtures thereof.

Specific antibiotics that may be used (alone or in combination) include, for example, amikacin, gentamicin, kanamycin, neomycin, netilmicin, streptomycin, tobramycin, paromomycin, geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin, azlocillin, carbenicillin, cloxacillin, dicloxacillin, flucloxacillin, mezlocillinm, meticillin, nafcillin, oxacillin, penicillin, piperacillin, ticarcillin, ciprofloxacin, enoxacin, gatifloxacin, levofloxacin, lomefloxacin, fodaximicin, moxifloxacin, norfloxacin, ofloxacin, trovafloxacin, grepafloxacin, sparfloxacin, temafloxacin, mafenide, sulfonamidochrysoidine sulfacetamide, sulfadiazine, ulfamethizole, sulfanilamide, sulfasalazine, sulfisoxazole, trimethoprim, trimethoprim-sulfamethoxazole (co-trimoxazole) (TMP-SMX), doxycycline, minocycline, oxytetracycline, tetracycline, chloramphenicol, clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristin/dalfopristin, rifampicin, thiamphenicol, tinidazole, dapsone, clofazimine and mixtures thereof.

In addition, the formulation may include a pharmaceutical agent that, while not formally classed as an antibiotic, acts to disrupt the normal ecology of the gastrointestinal tract and/or facilitate an increased proliferation of *C*. *difficle* and other harmful bacteria. Such agents may be included additively in the formulation or in place of the antibiotic.

In an embodiment, the formulation may include a combination of 2, of 3, of 4, of 5 or of 6 or more antibiotics or agents. The combination or single antibiotic(s) chosen will necessarily depend on the nature and type of infection that is to be treated in each circumstance and the medical particulars of the individual patient. Such selection is well within the scope of a person of skill in the art. In one embodiment, at least one of the several antibiotics includes fodaximicin.

The formulation also includes at least one probiotic material. "Probiotic material" means any material (including a neat culture) that contains live microorganisms (bacterial, viruses, and/or yeasts/fungi) of a type that have at least a minimal benefit to the human and/or mammalian digestive tract. Useful probiotics materials may include *Bacillus coagulans, Bifidobacterium animalis* subsp. *Lactis, Bifidobacterium breve, Bifidobacterium infantis, Bifidobacterium animalis, Bifidobacterium longum, Escherichia coli* M-17, *Escherichia coli* Nissle 1917, *Lactobacillus acidophilus, Lactobacillus casei, Lactobacillus paracasei, Lactobacillus fortis, Lactobacillus johnsonii, Lactococcus lactis, Lactobacillus plantarum, Lactobacillus Lactobacillus reuteri, Lactobacillus rhamnosus, Lactobacillus rhamnosus, Saccharomyces cerevisiae,* especially *boulardii, Lactobacillus rhamnosus, Streptococcus thermophilus, Lactobacillus helveticus,* mixtures thereof, and/or other bacteria of the above-listed genera.

In an embodiment, the formulation may include a combination of 2 to 10 or more probiotic materials. The specific combination of probiotic materials or single probiotic material chosen will depend on the nature and type of antibiotic(s) that are also included in the formulation and the medical particulars of the individual patient. Such selection is well within the scope of a person of skill in the art. In an embodiment, the selected probiotic material may be a blend of bacteria of the genus *Bifidobacterium,* of bacteria of the genus *Lactobacillus,* preferred may be species *paracasei* and/or *acidophilus,* and *Streptococcus thermophilus.* In an embodiment, the probiotic material includes *Saccharomyces cerevisiae (boulardii).*

The formulation may be prepared and administered in any dosage form suitable for administration to the gastrointestinal tract. In an embodiment, the dosage form is suitable for oral administration, such as, for example, a tablet, capsule, soft gel, chewie, and/or any other solid or semi solid dosage form. Such forms may optionally include polymers and /or other materials that permit extended release and /or targeted release to specific sites within the gastrointestinal tract. The oral dosage form may be a powder or granules that can be consumed neat or added to food or liquid.

In one embodiment, the probiotic material(s) and the antibiotic(s) are physically segregated within the dosage form, for example, separated by an acrylic or cellulose membrane in a tablet or capsule. In an alternative embodiment, the antibiotic(s) and the probiotic material(s) are in physical contact within the dosage form, such as for example, in a mixed powder or granules form (free or formed into a tablet or capsule) or a liquid. In an embodiment, the dosage form may be adapted for administration directly to the digestive tract, via a device or portal, such as, for example, a feeding tube.

Alternatively, the formulation may be prepared as a liquid or gel, a suspension, an emulsion, a solution, and a mixture.

The formulation may include any additional component as desired, as long as such components do not substantially erode the effectiveness of the antibiotic or the probiotic material. Such components may include, for example, thickeners, sweeteners, flavorants, fragrances, additional pharmaceuticals, glycine, mannitol, silicone dioxide, silica gels, binders, vitamins, carriers (such as, for example, glycerin, starches, celluloses, chitins, starches, water, alcohol) and minerals.

The unit dose and the dosage regimen of each the antibiotic(s) and the probiotic material(s) will be dictated by the specific antibiotic(s) and probiotic material(s) selected the dosage form and the medical particulars of the patient. In general, the unit dose and the dosage regimen will parallel that of the antibiotic if it were to be administered alone. For example, if the antibiotic recommended dosage is 200 mgs at two hour intervals, the antibiotic dosage in the formulation will remain substantially the same, as would the frequency of administration of the entire formulation. However, the therapeutically effective dosage of any specific antibiotic or agent will vary somewhat from antibiotic to antibiotic and/or probiotic material to probiotic material, patient to patient, and will depend upon the condition of the patient and the dosage form.

In general, the probiotic material per unit dose may be preferred to be at about 2 billion colony forming units (CFUs) to about 50 billion CFUs, about 4 billion CFUs to about 10 billion CFUs, or about 6 billion CFUs to about 8 billion CFUs, depending on the medical particulars of the patient, including age and weight.

Also include within the scope of the invention are methods of preventing or minimizing the occurrence of antibiotic-associated colitis in a patient undergoing antibiotic therapy. Such method includes administering the formulation to the patient's gastrointestinal tract (preferably orally) in the formulation described above, for the duration of therapy.

Methods of preventing or minimizing the occurrence of antibiotic-associated pseudomembranous colitis in a patient undergoing antibiotic therapy are also included. Such methods include administration of the formulations described above.

Methods of reducing or preventing a treatment failure of an antibiotic-treatable infection in a patient are described and include administration to the patient of a formulation of the invention. Antibiotic-treatable infections may include, for example, bacterial sinusitis, various sexually transmitted diseases of bacterial origin, pharyngitis, otitis, especially otitis media, bacterial bronchitis, bacterial pneumonia, diverticulitis, urinary tract infections, skin and skin structure infections, cellulitis, abscesses, furuncles, impetigo, pyoderma, wound infections, acne, nail infections, chronic bacterial prostatitis, acute pyelonephritis, and/or infections caused by any one or more of *Haemophilus influenzae, Haemophilus parainfluenzae, Moraxella catarrhalis, Staphylococcus atreus, Staphylococcus epidermidis, Staphylococcus saprophyticus, Pseudomonas aeruginosa, Serratia marcescens, Escherichia coli, Klebsiella pneumoniae, Streptococcus pneumoniae, Streptococcus neumoniaeparainfluenzae, Streptococcus pyogenes, Chlamydia pneumoniae, Legionella pneumophila, Mycoplasma pneumoniae, Vibrio cholerae, Bacillus anthracis, Eikenella corrodens, Neisseria gonorrhoeae, Enterococcus faecalis, Enterobacter cloacae, Proteus mirabilis,* bacteria of the genus *Bacteroides,* including *Bacteroides fragilis,* bacteria of the genus *Fusobacterium,* and bacteria of the genus *Peptostreptococcus,* and MRSA infections.

Also included are methods of treating *C*. *diffile* infections using the formulations described herein where the antibiotic includes at least a non-systemic gram negative antibiotic that is effective against C. difficile. In an embodiment, the antibiotic is fodaximicin and the probtiotic material includes a yeast, such as *Saccharomyces boulardii.* The method is effective in reducing the infection in a mammalian patient and treatment using the methods of the invention results in minimal reoccurrence of the C. *difficile* infection (e.g., a likelihood of reoccurrence of 70% or less, of 60% or less, of 50% or less, of 40% or less, of 30% or less, of 20% or less, of 10% or less, and/or 5% or less). By "reoccurrence" it is meant a development of the C. *difficile* infection within less than 60 days of the completion of the therapy.

### EXAMPLES

### EXAMPLE I:

A 78 year old patient is diagnosed with nosocomial pneumonia and is prescribed a course of levofloxicin and probiotic material to be administered in single dose units of 750mg levofloxicin and the equivalent of 8 CFUs probiotic materials every 24 hours for 14 days. A formulation of the invention is prepared by compounding 10.5 grams of levofloxicin with the equivalent of 112 CFUs of a probiotic material that is a blend of various bacteria of the genus *Lactobacillus,* including species *acidophilus.* The compounded mixture is divided into 14 equal aliquots; each is placed in a gelatin capsule. Accordingly, each filled capsule contains a single dose of 750mg levofloxicin and the equivalent of 8 CFUs of probiotic material.

The patient is fully compliant with the prescribed regimen. After 14 days, the patient's condition resolves and he suffers no adverse gastrointestinal effects during the course of treatment.

### EXAMPLE II:

A 5 year old patient is diagnosed with a middle ear infection. Her physician prescribes a course of AUGMENTIN (amoxicillin and potassium clavulanate) in single dosages of 250 mg amoxicillin/31.25 mg of clavulonic acid in a potassium salt to be administered twice a day for 10 days. AUGMENTIN is a trademark for an antibiotic formulation (active ingredients: amoxicillin/potassium clavulanate) available from GlaxoSmithKline, Philadelphia, Pennsylvania. Inactive ingredients of AUGMENTIN oral suspension (prepared) are water, colloidal silicon dioxide, flavorings, xanthan gum, and sweetener. Because the patient's physician is well aware of the high incidence of diarrhea associated with the use of AUGMENTIN, he prepares a formulation of the invention as follows.

A 100 milliliter suspension (having 250mg amoxicillin and 31.25 mg clavulonic acid in a potassium salt per5mL aqueous suspension) is prepared in accordance with the AUGMENTIN instructions. A second 100 ml suspension is prepared by suspending an amount of probiotic material (the blend used in Example I) equivalent to 80 CFUs in water. The first suspension and the second suspension are combined and placed in a suitable dispensing bottle.

For 10 days, the patient is administered 10 mLs of the formulation; each single dose is therefore 250mg amoxicillin, 31.25 mg clavulonic acid in a potassium salt, and 4 CFUs of probiotic material.

The patient is fully compliant with the prescribed regimen. After 10 days, the patient's condition resolves and she suffers no adverse gastrointestinal effects during the course of treatment.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

## Claims

1. A formulation for use in a method of treatment of a patient having a infection selected from bacterial sinusitis, a sexually transmitted diseases of bacterial origin, pharyngitis, otitis, otitis media, bacterial bronchitis, bacterial pneumonia, a urinary tract infection, and skin and skin structure infections, cellulitis, abscesses, furuncles, impetigo, pyoderma, wound infections, acne, nail infections, chronic bacterial prostatitis, and pyelonephritis while simultaneously preventing a Clostridium difficile infection of a gastrointestinal tract of the patient comprising administering said formulation to the gastrointestinal tract of the patient;
wherein said formulation comprises at least one antibiotic in an amount sufficient to be systemically therapeutically effective for the non-gastrointestinal tract infection of the patient and at least one probiotic material,
wherein the formulation is a dosage form for delivery to the gastrointestinal tract and the antibiotic and the probiotic material are in physical contact with one another in the formulation whereby the non-gastrointestinal tract infection is treated and the patient is prevented from developing a Clostridium difficile infection of the gastrointestinal tract.

2. The formulation of claim 1, wherein the dosage form is selected from a tablet, capsule, soft gel, chewy, and gel cap.

3. The formulation of claim 1, wherein the antibiotic is selected from aminoglycosides, ansamycins, carbacephemes, cephalosporins, glycopeptides, macrolides, penicillins, polypeptides, quinolones, sulfonamides, tetracyclines and mixtures thereof.

4. The formulation of claim 1, wherein the antibiotic is selected from amikacin, gentamicin, kanamycin, neomycin, netilmicin, streptomycin, fidaximicin, tobramycin, and paromomycin.

5. The formulation of claim 1, wherein the antibiotic is selected from geldanamycin, herbimycin, loracarbef, ertapenem, doripenem, imipenem/cilastatin, meropenem, cefadroxil, duricef, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftobiprole, teicoplanin, vancomycin, azithromycin, clarithromycin, dirithromycin, erythromycin, roxithromycin, troleandomycin, telithromycin, spectinomycin, aztreonam, amoxicillin, ampicillin and azlocillin.

6. The formulation of claim 1, wherein the antibiotic is selected from Carbenicillin, cloxacillin, dicloxacillin, flucloxacillin, mezlocillin, meticillin, nafcillin, oxacillin, penicillin, piperacillin, ticarcillin, ciprofloxacin, enoxacin, gatifloxacin, levofloxacin, lomefloxacin, moxifloxacin, norfloxacin, ofloxacin, trovafloxacin, grepafloxacin, sparfloxacin, temafloxacin, mafenide, sulfonamidochrysoidine, sulfacetamide, sulfadiazine, sulfamethizole, sulfanilimide, sulfasalazine, sulfisoxazole, trimethoprim, trimethoprim-sulfamethoxazole, and cotrimoxazole (TMP-SMX).

7. The formulation of claim 1, wherein the antibiotic is selected from doxycycline, minocycline, oxytetracycline, tetracycline, chloramphenicol, clindamycin, lincomycin, ethambutol, myambutol, fosfomycin, fusidic acid, furazolidone, isoniazid, linezolid, metronidazole, mupirocin, nitrofurantoin, platensimycin, pyrazinamide, quinupristinldalfopristin, rifampicin, thiamphenicol, tinidazole, dapsone and clofazimine.

8. The formulation of claim 1, wherein said formulation contains at least two antibiotics.

9. The formulation of claim 1, wherein the at least one probiotic material is selected from *Bacillus coagulans, Bifidobacterium animalis* subsp. *lactis, Bifidobacterium breve, Bifidobacterium infantis, Bifidobacterium animalis, Bifidobacterium longum, Escherichia coli* M-17, *Escherichia coli Nissle* 1917, *Lactobacillus acidophilus, Lactobacillus casei, Lactobacillus paracasei, Lactobacillus jortis, Lactobacillus johnsonii, Lactococcus lactis, Lactobacillus plantarum, Lactobacillus reuteri, Saccharomyces boulardii, Saccharomyces cerevisiae, Lactobacillus rhamnosus, Lactobacillus helveticus,* and mixtures thereof.

10. The formulation of claim 1, wherein the probiotic material is present in an amount of about 2 billion CFUs to about 50 billion CFUs, per each, single unit dose.

11. The formulation of claim 1, wherein the probiotic material is present in an amount of about 4 billion CFUs to about 10 billion CFUs per each single unit dose.

12. The formulation of claim 1, wherein the probiotic material is present in an amount of about 6 billion CFUs to about 8 billion CFUs, per each single unit dose.
